(19) Europäisches Patentamt
European Patent Office
Office européen des brevets

(11) **EP 4 424 415 A1**

(12) **EUROPEAN PATENT APPLICATION**

(43) Date of publication:
**04.09.2024 Bulletin 2024/36**

(21) Application number: **23382181.8**

(22) Date of filing: **28.02.2023**

(51) International Patent Classification (IPC):
**B01J 23/00** *(2006.01)*    **B01J 23/28** *(2006.01)*
**B01J 35/00** *(2024.01)*

(52) Cooperative Patent Classification (CPC):
(C-Sets available)
**B01J 23/002; B01J 23/28; B01J 35/30;**
B01J 2523/00    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Dow Global Technologies LLC
Midland, MI 48674 (US)**

(72) Inventors:
• **POLLEFEYT, Glenn
4542 NM Hoek (NL)**
• **RIVERA, Miguel
28760 Tres Cantos, Madrid (ES)**
• **McADON, Mark H.
Midland, Michigan, 48674 (US)**

• **KIRILIN, Alexey
4542 NM Hoek (NL)**
• **CHOJECKI, Adam
4542 NM Hoek (NL)**
• **BLANN, Kevin
Freeport, TX 77541 (US)**
• **MALEK, Andrzej
4542 NM Hoek (NL)**

(74) Representative: **Elzaburu S.L.P.
Edificio Torre de Cristal
Paseo de la Castellana 259 C, planta 28
28046 Madrid (ES)**

Remarks:
Amended claims in accordance with Rule 137(2)
EPC.

(54) **CATALYST AND PROCESS FOR THE DEHYDROGENATION OF ALKANES TO OLEFINS**

(57)    An oxidative dehydrogenation catalyst having: a structure having a formula $Mo_uV_vNb_wSb_yBi_zO_x$, where u is 1, v is from 0.1 to 0.5, w is from 0.001 to 0.3, y is from 0.001 to 0.2, z is from 0.03 to 0.2, and x is the oxygen content required to charge-balance the structure. The oxidative dehydrogenation catalyst comprises a crystalline structure (Pba2-32 space group) characterized by reflections determined with Cu-$K_\alpha$ X-ray diffraction (XRD) as follows:

| 2θ (± 0.3°) | Rel. Intensity (%) |
|---|---|
| 5.3 | 0.2 – 10 |
| 6.6 | 1.5 – 15 |
| 7.84 | 2.5 – 45 |
| 8.95 | 4 – 21 |
| 22.17 | 100 |
| 27.2 | 20 – 70 |

EP 4 424 415 A1

**(Cont. next page)**

(52) Cooperative Patent Classification (CPC): (Cont.)

C-Sets
B01J 2523/00, B01J 2523/53, B01J 2523/54, B01J 2523/55, B01J 2523/56, B01J 2523/68, B01J 2523/822

**Description**

**BACKGROUND**

*Field*

The present specification generally relates to catalysts for the dehydrogenation of alkanes to olefins, such as catalysts for converting ethane to ethylene.

*Technical Background*

Conventional catalysts for converting alkanes to olefins, such as converting ethane to ethylene and acetic acid, are based on molybdenum (Mo), vanadium (V), and niobium (Nb) and include promoters such as calcium (Ca), sodium (Na), antimony (Sb), or tellurium (Te). In particular, Te is a common promoter included in conventional catalysts. Processes using such catalysts require an oxygen co-feed and utilize an oxidative dehydrogenation process at low temperature, such as below 500 °C, and low pressures, such as below 300 pounds per square inch gauge (psig) (or about 20 barg).

**SUMMARY**

According to one embodiment, an oxidative dehydrogenation catalyst comprises: (i) a structure comprising oxides of molybdenum (Mo), vanadium (V), niobium (Nb), antimony (Sb), and bismuth (Bi) having a formula $Mo_uV_vNb_wSb_yBi_zO_x$, wherein u is 1, v is from 0.1 to 0.5, w is from 0.001 to 0.3, y is from 0.001 to 0.2, z is from 0.03 to 0.2, and x is the oxygen content required to charge-balance the structure, and (ii) a crystalline structure with the Pba2-32 space group, characterized by reflections determined with Cu-$K_\alpha$ X-ray diffraction (XRD) as follows

| 2θ (± 0.3°) | Rel. Intensity (%) |
|---|---|
| 5.3 | 0.2 - 10 |
| 6.6 | 1.5 - 15 |
| 7.84 | 2.5 - 45 |
| 8.95 | 4 - 21 |
| 22.17 | 100 |
| 27.2 | 20 - 70 |

According to another embodiment, a method for forming an oxidative dehydrogenation catalyst comprises: adding a molybdenum-containing compound, a vanadium-containing compound, a bismuth-containing compound, a niobium-containing compound, an antimony-containing compound, and one or more organic acids to a mixture of complexing agents and water ; synthesizing $Mo_uV_vNb_wSb_yBi_zO_x$ by hydrothermal synthesis at a hydrothermal synthesis temperature for a period of time, wherein u is 1, v is from 0.1 to 0.5, w is from 0.001 to 0.3, y is from 0.001 to 0.2, z is from 0.03 to 0.2, and x is the oxygen content required to charge-balance the structure; and separating the $Mo_uV_vNb_wSb_yBi_zO_x$ from retained liquids.

In another embodiment, a method for converting paraffins to olefins comprises: contacting a feed stream comprising paraffins with the oxidative dehydrogenation catalyst; converting at least a portion of the paraffins to olefins, thereby yielding a product stream comprising paraffins and olefins; and separating the olefins from the paraffins in the product stream, wherein the oxidative dehydrogenation catalyst comprises: (i) a structure comprising oxides of molybdenum (Mo), vanadium (V), niobium (Nb), antimony (Sb), and bismuth (Bi) having a formula $Mo_uV_vNb_wSb_yBi_zO_x$, wherein u is 1, v is from 0.1 to 0.5, w is from 0.001 to 0.3, y is from 0.001 to 0.2, z is from 0.03 to 0.2, and x is the oxygen content required to charge-balance the structure, and (ii) a crystalline structure (Pba2-32 space group) characterized by reflections determined with Cu-$K_\alpha$ X-ray diffraction (XRD) as follows:

| 2θ (± 0.3°) | Rel. Intensity (%) |
|---|---|
| 5.3 | 0.2 - 10 |
| 6.6 | 1.5 - 15 |

(continued)

| 2θ (± 0.3°) | Rel. Intensity (%) |
|---|---|
| 7.84 | 2.5 - 45 |
| 8.95 | 4 - 21 |
| 22.17 | 100 |
| 27.2 | 20 - 70 |

As would be recognized by one of ordinary skill in the art, the relative intensity may be affected by the preferential orientation effect and the above-disclosed relative intensities take into account such effects.

Additional features and advantages will be set forth in the detailed description that follows, and in part will be readily apparent to those skilled in the art from that description or recognized by practicing the embodiments described herein, including the detailed description which follows and the claims.

It is to be understood that both the foregoing general description and the following detailed description describe various embodiments and are intended to provide an overview or framework for understanding the nature and character of the claimed subject matter.

## BRIEF DESCRIPTION OF DRAWINGS

The following detailed description may be better understood when read in conjunction with the following drawings, in which:

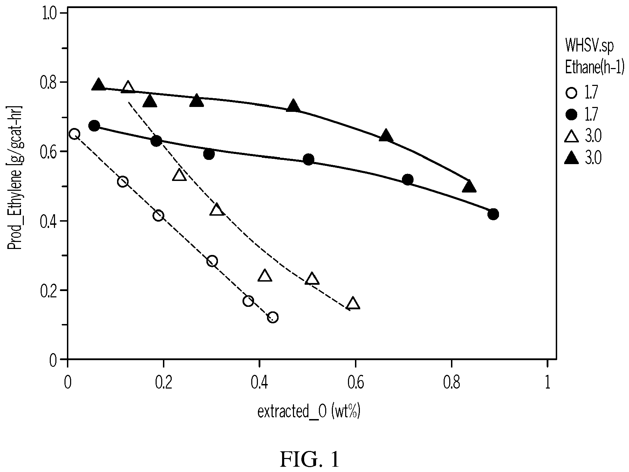

FIG 1. is a graph of ethylene productivity as a function of oxygen extracted from the catalyst at different space velocity conditions.

## DETAILED DESCRIPTION

Reference will now be made in detail to embodiments of catalysts for the dehydrogenation of alkanes to olefins, such as catalysts for converting ethane to ethylene and methods for making such catalysts.

One issue with conventional oxidative dehydrogenation processes is that they require a co-feed stream of oxygen ($O_2$). This adds costs to the process by requiring equipment that can produce pure, or nearly pure, oxygen to use in the process. In addition, the presence of oxygen in the process increases the chances for undesired, dangerous combustion as the oxygen and hydrocarbons mix. Conventional catalysts also require a certain amount of oxygen to be present for the catalyst to remain stable. Finally, because of the nature of the catalyst, and the oxygen requirement for dehydrogenation of alkanes, conventional oxidative dehydrogenation processes for converting alkanes to olefins are conducted in fixed bed reactors, which requires down time to remove and replace or regenerate the catalyst. Accordingly, there is a need for improved catalysts that can convert alkanes to olefins. While conventional catalysts may be used in oxidative dehydrogenation, they show limited stability when operated in a cyclic redox mode or in conditions with low partial pressure of $O_2$, which are typically encountered at the bottom sections of fixed bed reactors, because of chemical changes, or the reduction and loss of elements like tellurium.

In cyclic redox mode, one key aspect is that the oxygen capacity of the catalyst needs to be high enough (> 0.8 wt.%) in order to obtain a substantial per-pass ethane conversion (> 40%) at industrially feasible solid/ethane feed rates in various circulating bed reactor technologies. The previously disclosed MoVNbBiOx-type catalyst compositions typically have limited apparent oxygen capacity of about 0.4-0.5 wt.% of extractable lattice oxygen, which renders the previously disclosed MoVNbBiOx-type catalyst compositions incapable of delivering the required amount of oxygen for industrially feasible solid/ethane feed rates. On the other hand, catalysts of the MoVNbSbOx-type show higher oxygen capacities than the MoVNbBiOx-type catalyst compositions, but these MoVNbSbOx-type catalysts typically suffer from lower selectivity to ethylene (around 65%), thereby losing substantial amounts of its lattice oxygen to form CO and $CO_2$.

It has unexpectedly been found that the addition of small amounts of antimony (Sb) to the MoVNbBiOx-type materials disclosed and described herein can lead to a substantial increase in oxygen capacity, allowing for sustained ethane conversion at lower solid/ethane feed rates while maintaining high ethylene selectivity (> 75%). Upon the addition of Sb to the MoVNbBiOx-structure, the apparent oxygen capacity of the catalyst significantly increases from 0.4 wt.% to about 0.7 - 1.2 wt.% depending on the exact composition of the MoVNbSb,BiOx materials.

In one or more embodiments, the oxidative dehydrogenation catalyst has the following chemical formula: $Mo_u$-$V_vNb_wSb_yBi_zO_x$, where u is 1.0 (*e.g.,* Mo is used as the basis for the atomic ratios), v is from 0.1 to 0.5, w is from 0.001 to 0.3, y is from 0.001 to 0.2, z is from 0.03 to 0.2, and x is the oxygen content required to charge-balance the structure. In embodiments, v is from 0.2 to 0.5, from 0.3 to 0.5, from 0.4 to 0.5, from 0.1 to 0.4, from 0.2 to 0.4, from 0.3 to 0.4, from 0.1 to 0.3, from 0.2 to 0.3, or from 0.1 to 0.2. In embodiments, w is from 0.01 to 0.3, from 0.05 to 0.3, from 0.1 to 0.3, from 0.15 to 0.3, from 0.2 to 0.3, from 0.25 to 0.3, from 0.001 to 0.25, from 0.01 to 0.25, from 0.05 to 0.25, from 0.1 to 0.25, from 0.15 to 0.25, from 0.2 to 0.25, from 0.01 to 0.2, from 0.05 to 0.2, from 0.1 to 0.2, from 0.15 to 0.2, from 0.01 to 0.15, from 0.05 to 0.15, from 0.1 to 0.15, from 0.01 to 0.1, from 0.05 to 0.1, or from 0.01 to 0.05. In embodiments, y is from 0.001 to 0.2, from 0.001 to 0.01, from 0.001 to 0.1, from 0.001 to 0.05, from 0.001 to 0.005, from 0.01 to 0.1, from 0.05 to 0.1 from 0.001 to 0.002, or from 0.001 to 0.005. In embodiments, z is from 0.05 to 0.3, from 0.10 to 0.3, from 0.15 to 0.3, from 0.2 to 0.3, from 0.25 to 0.3, from 0.01 to 0.25, is from 0.05 to 0.25, from 0.10 to 0.25, from 0.15 to 0.25, from 0.2 to 0.25, from 0.01 to 0.2, is from 0.05 to 0.2, from 0.10 to 0.2, from 0.15 to 0.2, from 0.01 to 0.15, is from 0.05 to 0.15, from 0.10 to 0.15, from 0.01 to 0.1, is from 0.05 to 0.1, or from 0.01 to 0.05. In embodiments, the oxidative dehydrogenation catalyst has the following formula: $MoV_{0.2-0.3}Nb_{0.005-0.02}Sb_{0.05-0.15}Bi_{0.05-0.15}O_x$, where x is the oxygen content required to charge-balance the structure. It should be understood that embodiments of the $Mo_vV_wNb_yBi_zO_x$ catalyst having a Pba2-32 space that is essentially free of Te, such as having a Te/Mo atomic ratio that is less than or equal to 0.01.

The crystal structure of the oxidative dehydrogenation catalyst disclosed and described herein can, in embodiments, be measured using X-Ray Diffraction (XRD). For instance, and as would be understood by a skilled artisan, the position and relative intensity of XRD peaks at various $2\theta$ angles can be used to describe the crystal structure of the oxidative dehydrogenation catalyst. In embodiments, the oxidative dehydrogenation catalyst has reflections determined with Cu-K$_\alpha$ XRD as shown in Table 1. In Table 1 below, the relative intensity (Rel. Intensity) of the diffractogram features is the largest at $2\theta = 22.17°$ and, thus, this relative intensity is set to 100% and used as the basis for the remaining relative intensities shown in Table 1. As would be recognized by one of ordinary skill in the art, the relative intensity may be affected by the preferential orientation effect and the above-disclosed relative intensities take into account such effects.

**Table 1**

| $2\theta$ (± 0.3°) | Rel. Intensity (%) |
|---|---|
| 5.3 | 0.2 - 10 |
| 6.6 | 1.5 - 15 |
| 7.84 | 2.5 - 45 |
| 8.95 | 4 - 21 |
| 22.17 | 100 |
| 27.2 | 20 - 70 |

As mentioned above, using a specific hydrothermal method for forming the oxidative dehydrogenation catalyst allows the oxidative dehydrogenation catalysts to be formed having the desired Pba2-32 crystal structure. Embodiments of these hydrothermal methods for forming the oxidative dehydrogenation catalyst will now be described in more detail.

Oxidative dehydrogenation catalysts having a $Mo_uV_vNb_wSb_yBi_zO_x$ structure are, in one or more embodiments, formed through a synthetic process started by adding a molybdenum-containing compound, a vanadium-containing compound, a bismuth-containing compound, an antimony-containing compound, and a niobium-containing compound, and one or more organic acids to a mixture of alkylene glycol or alcohol amines and water to form a reaction mixture. In embodiments, the metal precursors are chosen such that the precursors can be dissolved/digested under hydrothermal reaction conditions. $Mo_uV_vNb_wSb_yBi_zO_x$ is then synthesized from the reaction mixture by hydrothermal synthesis at a hydrothermal synthesis temperature, which is described below, for a period of time. After the period of time has passed, $Mo_uV_vNb_wSb_yBi_zO_x$ is separated from retained liquids. In one or more embodiments, the molybdenum-containing compound, vanadium-containing compound, bismuth-containing compound, antimony-containing compound, niobium-containing compound, and one or more acids are added to the mixture of alkylene glycol and water, sequentially.

In embodiments, the bismuth-containing compound is selected from the group consisting of bismuth oxide ($Bi_2O_3$), bismuth sulfate ($Bi_2(SO_4)_3$), bismuth citrate ($BiC_6H_5O_7$), and bismuth nitrate ($Bi(NO_3)_3$). In embodiments, the niobium-containing compound is selected from the group consisting of niobium oxide, niobic acid ($Nb_2O_5 \cdot nH_2O$), niobium ethoxide, and ammonium niobium oxalate hydrate (($NH_4)Nb(C_2O_4)_2 \cdot nH_2O$). In embodiments, the molybdenum-containing compound can be ammonium heptamolybdate ($(NH_4)_6Mo_7O_{24}$ or molybdenum trioxide ($MoO_3$), and the vanadium-containing compound can be ammonium metavanadate ($NH_4VO_3$), vanadyl sulfate ($VOSO_4$), or vanadium pentoxide ($V_2O_5$). In embodiments, the antimony-containing compound is selected from the group consisting of antimony trioxide ($Sb_2O_3$) and antimony pentoxide ($Sb_2O_5$) The molybdenum-containing compound and the vanadium-containing compound are, in embodiments, $MoO_3$ and $V_2O_5$, respectively. In some embodiments, a digestible mixture of metal containing compounds having the correct stoichiometric ratio of one or more of Mo, V, Nb, Sb, and Bi could be used. Examples of such digestible mixtures include $(Mo,V)O_x$ and $BiNbO_x$, $SbVO_x$, and $BiMoO_x$. In one or more embodiments, the acid is selected from the group consisting of citric acid ($C_6H_8O_7$), oxalic acid ($C_2H_2O_4$), and mixtures thereof. In embodiments, the alkylene glycol is ethylene glycol.

The hydrothermal synthesis temperature is, in embodiments, from 150 °C to 250 °C, from 160 °C to 250 °C, from 170 °C to 250 °C, from 180 °C to 250 °C, from 190 °C to 250 °C, from 200 °C to 250 °C, from 210 °C to 250 °C, from 220 °C to 250 °C, from 230 °C to 250 °C, from 240 °C to 250 °C, from 150 °C to 240 °C, from 160 °C to 240 °C, from 170 °C to 240 °C, from 180 °C to 240 °C, from 190 °C to 240 °C, from 200 °C to 240 °C, from 210 °C to 240 °C, from 220 °C to 240 °C, from 230 °C to 240 °C, from 150 °C to 230 °C, from 160 °C to 230 °C, from 170 °C to 230 °C, from 180 °C to 230 °C, from 190 °C to 230 °C, from 200 °C to 230 °C, from 210 °C to 230 °C, from 220 °C to 230 °C, from 150 °C to 220 °C, from 160 °C to 220 °C, from 170 °C to 220 °C, from 180 °C to 220 °C, from 190 °C to 220 °C, from 200 °C to 220 °C, from 210 °C to 220 °C, from 150 °C to 210 °C, from 160 °C to 210 °C, from 170 °C to 210 °C, from 180 °C to 210 °C, from 190 °C to 210 °C, from 200 °C to 210 °C, from 150 °C to 200 °C, from 160 °C to 200 °C, from 170 °C to 200 °C, from 180 °C to 200 °C, from 190 °C to 200 °C, from 150 °C to 190 °C, from 160 °C to 190 °C, from 170 °C to 190 °C, from 180 °C to 190 °C, from 150 °C to 180 °C, from 160 °C to 180 °C, from 170 °C to 180 °C, from 150 °C to 170 °C, from 160 °C to 170 °C, or from 150 °C to 160 °C.

In embodiments, the hydrothermal pressure is from 4 bar (400 kPa) to 40 bar (4000 kPa), such as from 5 bar (500 kPa) to 40 bar (4000 kPa), from 10 bar (1000 kPa) to 40 bar (4000 kPa), from 15 bar (1500 kPa) to 40 bar (4000 kPa), from 20 bar (2000 kPa) to 40 bar (4000 kPa), from 25 bar (2500 kPa) to 40 bar (4000 kPa), from 30 bar (3000 kPa) to 40 bar (4000 kPa), from 35 bar (3500 kPa) to 40 bar (4000 kPa), from 4 bar (400 kPa) to 35 bar (3500 kPa), from 5 bar (500 kPa) to 35 bar (3500 kPa), from 10 bar (1000 kPa) to 35 bar (3500 kPa), from 15 bar (1500 kPa) to 35 bar (3500 kPa), from 20 bar (2000 kPa) to 35 bar (3500 kPa), from 25 bar (2500 kPa) to 35 bar (3500 kPa), from 30 bar (3000 kPa) to 35 bar (3500 kPa), from 4 bar (400 kPa) to 30 bar (3000 kPa), from 5 bar (500 kPa) to 30 bar (3000 kPa), from 10 bar (1000 kPa) to 30 bar (3000 kPa), from 15 bar (1500 kPa) to 30 bar (3000 kPa), from 20 bar (2000 kPa) to 30 bar (3000 kPa), from 25 bar (2500 kPa) to 30 bar (3000 kPa), from 4 bar (400 kPa) to 25 bar (2500 kPa), from 5 bar (500 kPa) to 25 bar (2500 kPa), from 10 bar (1000 kPa) to 25 bar (2500 kPa), from 15 bar (1500 kPa) to 25 bar (2500 kPa), from 20 bar (2000 kPa) to 25 bar (2500 kPa), from 4 bar (400 kPa) to 20 bar (2000 kPa), from 5 bar (500 kPa) to 20 bar (2000 kPa), from 10 bar (1000 kPa) to 20 bar (2000 kPa), from 15 bar (1500 kPa) to 20 bar (2000 kPa), from 4 bar (400 kPa) to 15 bar (1500 kPa), from 5 bar (500 kPa) to 15 bar (1500 kPa), from 10 bar (1000 kPa) to 15 bar (1500 kPa), from 4 bar (400 kPa) to 10 bar (1000 kPa), or from 5 bar (500 kPa) to 10 bar (1000 kPa).

According to embodiments, after the $Mo_uV_vNb_wSb_yBi_zO_x$ oxidative dehydrogenation catalyst is separated from the retained liquids, the $Mo_uV_vNb_wSb_yBi_zO_x$ oxidative dehydrogenation catalyst is dried and optionally calcined by heating the dried $Mo_uV_vNb_wSb_yBi_zO_x$ oxidative dehydrogenation catalyst to a calcination temperature and holding the $Mo_uV_vNb_wSb_yBi_zO_x$ oxidative dehydrogenation catalyst at the calcination temperature for a period of time.

In embodiments, the $Mo_uV_vNb_wSb_yBi_zO_x$ oxidative dehydrogenation catalyst may be dried at any suitable temperature. However, to expedite drying, the $Mo_uV_vNb_wSb_yBi_zO_x$ oxidative dehydrogenation catalyst may, in embodiments, be dried at temperatures from 65 °C to 200 °C, from 75 °C to 200 °C, from 100 °C to 200 °C, from 125 °C to 200 °C, from 150 °C to 200 °C, from 175 °C to 200 °C, 65 °C to 175 °C, from 75 °C to 175 °C, from 100 °C to 175 °C, from 125 °C to 175 °C, from 150 °C to 175 °C, 65 °C to 150 °C, from 75 °C to 150 °C, from 100 °C to 150 °C, from 125 °C to 150 °C, 65 °C to 125 °C, from 75 °C to 125 °C, from 100 °C to 125 °C, 65 °C to 100 °C, from 75 °C to 100 °C, or 65 °C to 75 °C.

In embodiments, the calcination takes place in an inert atmosphere, such as nitrogen ($N_2$), argon (Ar), or helium (He). In such embodiments, the calcination temperature is from 350 °C to 650 °C, from 375 °C to 650 °C, 400 °C to 650 °C, from 425 °C to 650 °C, from 450 °C to 650 °C, from 475 °C to 650 °C, from 500 °C to 650 °C, from 525 °C to 650 °C, from 550 °C to 650 °C, from 575 °C to 650 °C, from 600 °C to 650 °C, from 625 °C to 650 °C, from 350 °C to 625 °C, from 375 °C to 625 °C, from 400 °C to 625 °C, from 425 °C to 625 °C, from 450 °C to 625 °C, from 475 °C to 625 °C, from 500 °C to 625 °C, from 525 °C to 625 °C, from 550 °C to 625 °C, from 575 °C to 625 °C, from 600 °C to 625 °C, from 350 °C to 600 °C, from 375 °C to 600 °C, from 400 °C to 600 °C, from 425 °C to 600 °C, from 450 °C to 600 °C, from 475 °C to 600 °C, from 500 °C to 600 °C, from 525 °C to 600 °C, from 550 °C to 600 °C, from 575 °C to 600 °C, from 350 °C to 575 °C, from 375 °C to 575 °C, from 400 °C to 575 °C, from 425 °C to 575 °C, from 450 °C to 575 °C, from 475 °C to 575 °C, from 500 °C to 575 °C, from 525 °C to 575 °C, from 550 °C to 575 °C, from 350 °C to 550 °C, from 375 °C to 550 °C, from 400 °C to 550 °C, from 425 °C to 550 °C, from 450 °C to 550 °C, from 475 °C to 550 °C, from 500 °C to 550 °C, from 525 °C to 550 °C, from 350 °C to 525 °C, from 375 °C to 525 °C, from 400 °C to 525 °C, from 425 °C to 525 °C, from 450 °C to 525 °C, from 475 °C to 525 °C, from 500 °C to 525 °C, from 350 °C to 500 °C, from 375 °C to 500 °C, from 400 °C to 500 °C, from 425 °C to 500 °C, from 450 °C to 500 °C, from 475 °C to 500 °C, from 350 °C to 475 °C, from 375 °C to 475 °C, from 400 °C to 475 °C, from 425 °C to 475 °C, from 450 °C to 475 °C, from 350 °C to 450 °C, from 375 °C to 450 °C, from 400 °C to 450 °C, from 425 °C to 450 °C, from 350 °C to 425 °C, from 375 °C to 425 °C, from 400 °C to 425 °C. from 350 °C to 400 °C, from 375 °C to 400 °C, or from 350 °C to 375 °C.

In embodiments, the calcination takes place in air. In such embodiments, the calcination temperature may be from 200 °C to 500 °C, from 200 °C to 500 °C, from 400 °C to 500 °C, from 425 °C to 500 °C, from 450 °C to 500 °C, from 475 °C to 500 °C, from 350 °C to 475 °C, from 375 °C to 475 °C, from 400 °C to 475 °C, from 425 °C to 475 °C, from 450 °C to 475 °C, from 350 °C to 450 °C, from 375 °C to 450 °C, from 400 °C to 450 °C, from 425 °C to 450 °C, from 350 °C to 425 °C, from 375 °C to 425 °C, from 400 °C to 425 °C. from 350 °C to 400 °C, from 375 °C to 400 °C, or from 350 °C to 375 °C.

After the $Mo_uV_vNb_wSb_yBi_zO_x$ oxidative dehydrogenation catalyst has been formed, the $Mo_uV_vNb_wSb_yBi_zO_x$ oxidative dehydrogenation catalyst may be used in processes for converting alkanes in an alkane-containing feed stream to olefins. The processes disclosed and described herein may provide improved olefin selectivity by the $Mo_uV_vNb_wSb_y$-$Bi_zO_x$ oxidative dehydrogenation catalyst as time on stream increases. Processes disclosed and described herein generally include contacting a feed stream comprising alkanes (paraffins) with a material comprising the $Mo_uV_vNb_wSb_yBi_zO_x$ oxidative dehydrogenation catalyst in a reaction zone, converting at least a portion of the alkanes to olefins yielding a product stream comprising paraffins and olefins. It should be understood that the oxidative dehydrogenation catalyst may be used alone or with other additives. The reaction zone is not particularly limited and any type of reactor allowing for cyclic or continuous operation of the process may be used in embodiments. In embodiments, the reaction zone can be a fixed bed reactor, a fluidized bed reactor, a moving bed reactor, a bubbling bed reactor, circulating fluidized reactor, reverse flow reactor, or an ebullated bed reactor (each optionally with an oxygen co-feed). The reaction zone is not particularly limited to a single reaction zone and can consist of multiple reactors in either series or parallel configuration. Finally, the paraffins and olefins in the product stream are separated, the paraffins may be recycled back to the feed stream, and the olefins are used in downstream systems or as materials in various products and processes. Processes according to embodiments disclosed and described herein will be provided in more detail below.

According to embodiments, a feed stream is fed into a reaction zone, the feed stream comprises at least one alkane. In embodiments, the feed stream may be comprised entirely of alkane (e.g., 100% alkane). In one or more embodiments, the feed stream may contain oxygen, steam and/or inert gas. In embodiments, the feed stream comprises from 30 volume percent (vol%) to 90 vol% alkane, from 35 vol% to 90 vol% alkane, from 40 vol% to 90 vol% alkane, from 45 vol% to 90 vol% alkane, from 50 vol% to 90 vol% alkane, from 55 vol% to 90 vol% alkane, from 60 vol% to 90 vol% alkane, from 65 vol% to 90 vol% alkane, from 70 vol% to 90 vol% alkane, from 75 vol% to 90 vol% alkane, from 80 vol% to 90 vol% alkane, from 85 vol% to 90 vol% alkane, from 30 vol% to 85 vol% alkane, from 35 vol% to 85 vol% alkane, from 40 vol% to 85 vol% alkane, from 45 vol% to 85 vol% alkane, from 50 vol% to 85 vol% alkane, from 55 vol% to 85 vol% alkane, from 60 vol% to 85 vol% alkane, from 65 vol% to 85 vol% alkane, from 70 vol% to 85 vol% alkane, from 75 vol% to 85 vol% alkane, from 80 vol% to 85 vol% alkane, from 30 vol% to 80 vol% alkane, from 35 vol% to 80 vol% alkane, from 40 vol% to 80 vol% alkane, from 45 vol% to 80 vol% alkane, from 50 vol% to 80 vol% alkane, from 55 vol% to 80 vol% alkane, from 60 vol% to 80 vol% alkane, from 65 vol% to 80 vol% alkane, from 70 vol% to 80 vol% alkane, from 75 vol% to 80 vol% alkane, from 30 vol% to 75 vol% alkane, from 35 vol% to 75 vol% alkane, from 40 vol% to 75 vol% alkane, from 45 vol% to 75 vol% alkane, from 50 vol% to 75 vol% alkane, from 55 vol% to 75 vol% alkane, from 60 vol% to 75 vol% alkane, from 65 vol% to 75 vol% alkane, from 70 vol% to 75 vol% alkane, from 30 vol% to 70 vol% alkane, from 35 vol% to 70 vol% alkane, from 40 vol% to 70 vol% alkane, from 45 vol% to 70 vol% alkane, from 50 vol% to 70 vol% alkane, from 55 vol% to 70 vol% alkane, from 60 vol% to 70 vol% alkane, from 65 vol% to 70 vol% alkane, from 30 vol% to 65 vol% alkane, from 35 vol% to 65 vol% alkane, from 40 vol% to 65 vol% alkane, from 45 vol% to 65 vol% alkane, from 50 vol% to 65 vol% alkane, from 55 vol% to 65 vol% alkane, from 60 vol% to 65 vol% alkane, from 30 vol% to 60 vol% alkane, from 35 vol% to 60 vol% alkane, from 40 vol% to 60 vol% alkane, from 45 vol% to 60 vol% alkane, from 50 vol% to 60 vol% alkane, from 55 vol% to 60 vol% alkane, from 30 vol% to 55 vol% alkane, from 35 vol% to 55 vol% alkane, from 40 vol% to 55 vol% alkane, from 45 vol% to 55 vol% alkane, from 50 vol% to 55 vol% alkane, from 30 vol% to 50 vol% alkane, from 35 vol% to 50 vol% alkane, from 40 vol% to 50 vol% alkane, from 45 vol% to 50 vol% alkane, from 30 volume percent (vol%) to 45 vol% alkane, from 35 vol% to 45 vol% alkane, from 40 vol% to 45 vol% alkane, from 30 vol% to 40 vol% alkane, from 35 vol% to 40 vol% alkane, or from 30 vol% to 35 vol% alkane.

In embodiments, the at least one alkane is selected from the group consisting of ethane, propane, and combinations thereof. In embodiments, the inert gas is selected from the group consisting of nitrogen, $CO_2$, and combination thereof.

According to one or more embodiments, the weight ratio of the $Mo_uV_vNb_wSb_yBi_zO_x$ oxidative dehydrogenation catalyst in the reaction zone to alkane in the reaction zone is from 250 to 10, from 225 to 10, from 200 to 10, from 175 to 10, from 150 to 10, from 125 to 10, from 100 to 10, from 75 to 10, from 50 to 10, from 25 to 10, from 250 to 25, from 225 to 25, from 200 to 25, from 175 to 25, from 150 to 25, from 125 to 25, from 100 to 25, from 75 to 25, from 50 to 25, from 250 to 50, from 225 to 50, from 200 to 50, from 175 to 50, from 150 to 50, from 125 to 50, from 100 to 50, from 75 to 50, from 250 to 75, from 225 to 75, from 200 to 75, from 175 to 75, from 150 to 75, from 125 to 75, from 100 to 75, from 250 to 100, from 225 to 100, from 200 to 100, from 175 to 100, from 150 to 100, from 125 to 100, from 250 to 125, from 225 to 125, from 200 to 125, from 175 to 125, from 150 to 125, from 250 to 150, from 225 to 150, from 200 to 150, from 175 to 150, from 250 to 175, from 225 to 175, from 200 to 175, from 250 to 200, from 225 to 200, or from 250 to 225. In embodiments where the reaction zone in a fluidized bed catalyst or the like, the catalyst to alkane ratio is controlled by the mass feed rate of alkane and the mass feed rate of catalyst to the reaction zone.

In embodiments, the feed stream is essentially free from oxygen, meaning that the feed stream comprises less than 2.0 volume percent (vol%) oxygen, less than 1.5 vol% oxygen, or less than 0.5 vol% oxygen. In one or more embodiments, the feed stream is free of oxygen.

In one or more embodiments, an oxygen stream is added to the reaction zone. The concentration of oxygen in the oxygen stream is not particularly limited. For example, the oxygen concentration in the oxygen stream may be from 0.1 vol% to 99.9 vol%, such as from 5.0 vol% to 95.0 vol%, from 10.0 vol% to 90.0 vol%, from 15.0 vol% to 85.0 vol%, from 20.0 vol% to 80.0 vol%, from 25.0 vol% to 75.0 vol%, from 30.0 vol% to 70.0 vol%, from 35.0 vol% to 65.0 vol%, from 40.0 vol% to 60.0 vol%, or from 45.0 vol% to 55.0 vol%. In one or more embodiments, the concentration of oxygen in the oxygen stream is relatively low, such as from 0.1 vol% to 5.0 vol%, from 0.2 vol% to 5.0 vol%, from 0.5 vol% to 5.0 vol%, from 0.8 vol% to 5.0 vol%, from 1.0 vol% to 5.0 vol%, from 1.2 vol% to 5.0 vol%, from 1.5 vol% to 5.0 vol%, from 1.8 vol% to 5.0 vol%, from 2.0 vol% to 5.0 vol%, from 2.2 vol% to 5.0 vol%, from 2.5 vol% to 5.0 vol%, from 2.8 vol% to 5.0 vol%, from 3.0 vol% to 5.0 vol%, from 3.2 vol% to 5.0 vol%, from 3.5 vol% to 5.0 vol%, from 3.8 vol% to 5.0 vol%, from 4.0 vol% to 5.0 vol%, from 4.2 vol% to 5.0 vol%, from 4.5 vol% to 5.0 vol%, or from 4.8 vol% to 5.0 vol%. In embodiments, the oxygen stream may be air, which generally has an oxygen concentration of about 21.0 vol%.

The oxygen stream may, in embodiments, be added to the reaction zone sequentially to the feed stream, such that the feed stream and the oxygen stream are not added to the reaction zone at the same time. It should be understood that in embodiments, the oxygen stream may be added at various points within the reaction process. This may be accomplished by introducing the oxygen stream into the reaction zone at different locations within the reaction zone and/or introducing the oxygen stream at different time periods while the reaction is taking place.

In one or more embodiments, the oxygen stream is added to the reaction zone simultaneously to the feed stream. In such embodiments, the volume ratio of oxygen (in the oxygen stream) to alkanes (in the feed stream) in the reaction zone is from greater 0.0 to 1.0, from 0.1 to 1.0, from 0.2 to 1.0, from 0.3 to 1.0, from 0.4 to 1.0, from 0.5 to 1.0, from 0.6 to 1.0, from 0.7 to 1.0, from 0.8 to 1.0, from 0.9 to 1.0, or from greater than 0.0 to 0.2, from 0.0 to 0.3, from 0.0 to 0.4, from 0.0 to 0.5, from 0.0 to 0.6, from 0.0 to 0.7, from 0.0 to 0.8, or from 0.0 to 0.9.

The feed stream is contacted with the $Mo_uV_vNb_wSb_yBi_zO_x$ oxidative dehydrogenation catalyst as disclosed and described herein in the reaction zone under reaction conditions sufficient to form a product stream comprising olefins. The reaction conditions comprise a temperature within the reaction zone ranging, according to one or more embodiments, the temperature within the reaction zone is from 300 °C to 700 °C, from 350 °C to 700 °C, from 400 °C to 700 °C, from 450 °C to 700 °C, from 500 °C to 700 °C, from 550 °C to 700 °C, from 600 °C to 700 °C, from 650 °C to 700 °C, from 300 °C to 650 °C, from 350 °C to 650 °C, from 400 °C to 650 °C, from 450 °C to 650 °C, from 500 °C to 650 °C, from 550 °C to 650 °C, from 600 °C to 600 °C, from 300 °C to 600 °C, from 350 °C to 600 °C, from 400 °C to 600 °C, from 450 °C to 600 °C, from 500 °C to 600 °C, from 550 °C to 600 °C, from 300 °C to 550 °C, from 350 °C to 550 °C, from 400 °C to 550 °C, from 450 °C to 550 °C, from 500 °C to 550 °C, from 300 °C to 500 °C, from 350 °C to 500 °C, from 400 °C to 500 °C, from 450 °C to 500 °C, from 300 °C to 450 °C, from 350 °C to 450 °C, from 400 °C to 450 °C, from 300 °C to 400 °C, from 350 °C to 400 °C, or from 300 °C to 350 °C.

The reaction conditions also, in embodiments, include a pressure inside the reaction zone from 0 barg (0 KPa) to 20 barg (2000 KPa), from 5 barg (500 KPa) to 20 barg (2000 KPa), from 10 barg (1000 KPa) to 20 barg (2000 KPa), from 15 barg (1500 KPa) to 20 barg (2000 KPa), from 0 barg (0 KPa) to 15 barg (1500 KPa), form 5 barg (500 KPa) to 15 barg (1500 KPa), from 10 barg (1000 KPa) to 15 barg (1500 KPa), from 0 barg (0 KPa) to 10 barg (1000 KPa), form 5 barg (500 KPa) to 10 barg (1000 KPa), or from 0 barg (0 KPa) to 5 barg (500 KPa).

According to embodiments, the weight hour space velocity (WHSV) of the alkane feed stream and, optionally, the oxygen stream within the reaction zone is from 0.1 per hour (/h) to 10.0/h, from 0.5/h to 10.0/h, from 1.0/h to 10.0/h, from 2.0/h to 10.0/h, from 3.0/h to 10.0/h, from 4.0/h to 10.0/h, from 5.0/h to 10.0/h, from 6.0/h to 10.0/h, from 7.0/h to 10.0/h, from 8.0/h to 10.0/h, from 9.0/h to 10.0/h, 0.1/h to 9.0/h, from 0.5/h to 9.0/h, from 1.0/h to 9.0/h, from 2.0/h to 9.0/h, from 3.0/h to 9.0/h, from 4.0/h to 9.0/h, from 5.0/h to 9.0/h, from 6.0/h to 9.0/h, from 7.0/h to 9.0/h, from 8.0/h to 9.0/h, 0.1/h to 8.0/h, from 0.5/h to 8.0/h, from 1.0/h to 8.0/h, from 2.0/h to 8.0/h, from 3.0/h to 8.0/h, from 4.0/h to 8.0/h, from 5.0/h to 8.0/h, from 6.0/h to 8.0/h, from 7.0/h to 8.0/h, 0.1/h to 7.0/h, from 0.5/h to 7.0/h, from 1.0/h to 7.0/h, from 2.0/h to 7.0/h, from 3.0/h to 7.0/h, from 4.0/h to 7.0/h, from 5.0/h to 7.0/h, from 6.0/h to 7.0/h, 0.1/h to 6.0/h, from 0.5/h to 6.0/h, from 1.0/h to 6.0/h, from 2.0/h to 6.0/h, from 3.0/h to 6.0/h, from 4.0/h to 6.0/h, from 5.0/h to 6.0/h, 0.1/h to 5.0/h, from 0.5/h to 5.0/h, from 1.0/h to 5.0/h, from 2.0/h to 5.0/h, from 3.0/h to 5.0/h, from 4.0/h to 5.0/h, 0.1/h to 4.0/h, from 0.5/h to 4.0/h, from 1.0/h to 4.0/h, from 2.0/h to 4.0/h, from 3.0/h to 4.0/h, 0.1/h to 3.0/h, from 0.5/h to 3.0/h, from 1.0/h to 3.0/h, from 2.0/h to 3.0/h, 0.1/h to 2.0/h, from 0.5/h to 2.0/h, from 1.0/h to 2.0/h, 0.1/h to 1.0/h, from 0.5/h to 1.0/h, or 0.1/h to 0.5/h.

**EXAMPLES**

**EXAMPLE 1**

A mixture of 34 mL of $H_2O$ and 80 microliter of ethylene glycol was added to a 45 mL Teflon-insert autoclave (Model 4744 General Purpose Acid Digestion Vessel, Parr). While stirring, 2.7126g of MoOs, 0.0.3427g of $V_2O_5$, 0.4373g of $Bi_2O_3$, 0.0421g of $(NH_4)Nb(C_2O_4)_2$ . $xH_2O$, 0.1366g of $Sb_2O_3$, 0.2711g of Citric acid and 0.4775g of oxalic acid was added sequentially and stirred for 10 min. Hydrothermal synthesis of $MoV_{0.2}Nb_{0.005}Bi_{0.1}Sb_{0.01}Ox$ was performed in a rotating shaft oven at 190 °C, 48 hours, 10 rpm. The obtained material from the hydrothermal synthesis was purified using 200 mL of deionized water using vacuum filtration and subsequently dried at 85 °C overnight.
After drying, the material was calcined at 450 °C (at a heating rate of 2 °C/min) under a flow of $N_2$, for 2 hours. The material was compacted under 7-ton pressure and crushed and sieved to 40-80 mesh prior to loading in the reactor. Reaction conditions will be discussed in detail below.

**COMPARATIVE EXAMPLE 1**

A mixture of 34 mL of $H_2O$ and 160 microliter of ethylene glycol was added to a 45 mL Teflon-insert autoclave (Model 4744 General Purpose Acid Digestion Vessel, Parr). While stirring, 2.7126 g of $MoO_3$, 0.5141 g of $V_2O_5$, 0.4373 g of $Bi_2O_3$, 0.0842 g of $(NH_4)Nb(C_2O_4)_2$ . $xH_2O$, 0.5422 g of Citric acid and 0.2388 g of oxalic acid was added sequentially and stirred for 10 min. Hydrothermal synthesis of $MoV_{0.3}Nb_{0.01}Bi_{0.1}Ox$ was performed in a rotating shaft oven at 190 °C, 48 hours, 10 rpm. The obtained material from the hydrothermal synthesis was purified using 200 mL of deionized water using vacuum filtration and subsequently dried at 85 °C overnight.
After drying, the material was calcined at 450 °C (at a heating rate of 2 °C/min) under a flow of $N_2$, for 2 hours. The material was compacted under 7-ton pressure and crushed and sieved to 40-80 mesh prior to loading in the reactor. Reaction conditions will be discussed in detail below.

**REACTOR TESTING**

Performance testing was performed in a fixed-bed reactor set-up from SINTEF (High Pressure Reactor Assembly Module), with SS316 reactor tubes (I.D. 3 mm). For catalytic testing, 250 - 350 mg of 40-80 mesh catalyst particles were loaded in the reactors, and the reactors were operated in cyclic mode in which periods of ethane exposure were alternated with inert gas purge and oxidative regeneration at the desired temperature:
A lattice oxidative dehydrogenation (LODh) step used a 15-20 mL/min flow of 50 vol.% Ethane in helium at a pressure of 2.5 bar (absolute) and a WHSV of 1.7 - 3.0/hr.
A regeneration step used a 10 mL/min flow of 2.5 vol.% $O_2$ in helium at a pressure of 2.5 bar (absolute).
The reactor effluent composition was obtained by gas chromatography (GC) and the conversion and carbon-based selectivities are calculated using the following equations:

$$XC_2H_6 \ (\%) = [(\eta C_2H_6, \ in - \eta C_2H_6, \ out / \ \eta C_2H_6, \ in] \cdot 100; \tag{1}$$

and

$$S_j \ (\%) = [\alpha j \cdot \eta j, \ out / \ \sum \alpha j \cdot \eta j, out] \cdot 100 \tag{2}$$

where $XC_2H_6$ is defined as the $C_2H_6$ conversion (%), $\eta$, in is defined as the molar inlet flow of the component (mol/min), $\eta$, out is the molar outlet flow of the component (mol/min), $S_j$ is defined as the carbon based selectivity to product j (%), $\alpha j$ is the number of carbon atoms for product j. Carbon balance for all experiments was within 99-102%.
The catalyst/ethane ratio (g/g) is calculated based on the time-on-stream (TOS, min) in which the GC analyzes the reactor effluent:

$$Cat/ethane = w \ / \ (TOS \cdot \eta C_2H_6, \ in \cdot MW_{C2H6})$$

(3)

where w is defined as the catalyst mass, $\eta C_2H_6$, in is the molar inlet flow of ethane (mol/min) and $MW_{C2H6}$ is the molecular weight of ethane (30 g/mol).

I

The ethylene productivity (g/g cat/h) is calculated using the following equation:

$$C_2H_4 \ Productivity = [\eta C_2H_6, \ in * 60 * XC_2H_6 * S_{C2H4}/100)* MW_{C2H4}]/w$$

where $MW_{C2H4}$ is defined as the molecular weight of ethylene (28 g/mol).

The results of the catalytic testing at 450 °C are shown in FIG. 1, plotting ethylene productivity as a function of oxygen extracted from the catalyst at different space velocity conditions.

It can be observed that the composition of example 1 (solid line) is able to maintain much higher ethylene productivity rates over a wider range of extracted lattice oxygen compared to the Sb-free composition of comparative example 1 (dashed lines), for both low and high space velocity conditions. As can be observed from the dashed line, the catalyst productivity gets reduced to very low levels (<0.2 kg/kg cat-hr) when more than 0.4-0.5wt%. of lattice oxygen is extracted, irrespective of the space velocity. Catalyst according to the current invention are able to maintain higher productivity levels (>0.5 kg/kg cat/hr) even when already >0.8 wt.% of O has been removed from the catalyst. This highlights the superior oxygen capacity and availability of Sb-promoted $MoVNbBiO_x$-materials.

It will be apparent to those skilled in the art that various modifications and variations can be made to the embodiments described herein without departing from the spirit and scope of the claimed subject matter. Thus, it is intended that the specification cover the modifications and variations of the various embodiments described herein provided such modification and variations come within the scope of the appended claims and their equivalents.

**Claims**

1. An oxidative dehydrogenation catalyst comprising:

   (i) a structure comprising a mixed oxide of molybdenum (Mo), vanadium (V), niobium (Nb), antimony (Sb) and bismuth (Bi) having a formula $Mo_uV_vNb_wSb_yBi_zO_x$, wherein u is 1, v is from 0.1 to 0.5, w is from 0.001 to 0.3, y is from 0.001 to 0.2, z is from 0.03 to 0.2, and x is an oxygen content required to charge-balance the structure; and
   (ii) a crystallographic structure with the Pba2-32 space group, **characterized by** reflections determined with Cu-K$_\alpha$ X-ray diffraction (XRD) as follows:

| 2θ (± 0.3°) | Rel. Intensity (%) |
|---|---|
| 5.3 | 0.2 - 10 |
| 6.6 | 1.5 - 15 |
| 7.84 | 2.5 - 45 |
| 8.95 | 4 - 21 |
| 22.17 | 100 |
| 27.2 | 20 - 70 |

2. The oxidative dehydrogenation catalyst of claim 1, wherein the oxidative dehydrogenation catalyst consists of the structure comprising oxides of molybdenum (Mo), vanadium (V), niobium (Nb), antimony (Sb) and bismuth (Bi) having a formula $Mo_uV_vNb_wSb_yBi_zO_x$, wherein u is 1, v is from 0.1 to 0.5, w is from 0.001 to 0.3, y is from 0.001 to 0.1, and z is from 0.03 to 0.2, and x is the oxygen content required to charge-balance the structure.

3. The oxidative dehydrogenation catalyst of claim 1, wherein the oxidative dehydrogenation catalyst is essentially free of tellurium (Te).

4. A method for forming an oxidative dehydrogenation catalyst, the method comprising:

a) adding a molybdenum-containing compound, a vanadium-containing compound, a bismuth-containing compound, a niobium-containing compound, an antimony-containing compound, and one or more organic acids to a mixture of alkylene glycol or alcohol amine and water to form a starting mixture;
b) treating the starting mixture by hydrothermal synthesis at a hydrothermal synthesis temperature that is from 150 °C to 250 °C; and
c) separating $Mo_uV_vNb_wSb_yBi_zO_x$ from retained liquids,

wherein the starting mixture comprises at least one of molybdenum trioxide (MoOs) and vanadium pentoxide ($V_2O_5$).

5. The method for forming an oxidative dehydrogenation catalyst of claim 4, wherein the one or more organic acids comprises at least one of citric acid ($C_6H_8O_7$), oxalic acid ($C_2H_2O_4$), and mixtures thereof.

6. The method for forming an oxidative dehydrogenation catalyst of any one of claim 4 or claim 5, wherein the niobium-containing compound is selected from the group consisting of niobium oxide, niobic acid ($Nb_2O_5 \cdot xH_2O$), ammonium niobium oxalate hydrate($(NH_4)Nb(C_2O_4)_2.nH_2O$), niobium ethoxide, and mixtures thereof.

7. The method for forming an oxidative dehydrogenation catalyst of any one of claim 4 to 6, wherein the bismuth-containing compound is selected from the group consisting of bismuth oxide ($Bi_2O_3$), bismuth sulfate ($Bi_2(SO_4)_3$), bismuth citrate ($BiC_6H_5O_7$), and bismuth nitrate ($Bi(NO_3)_3$).

8. The method for forming an oxidative dehydrogenation catalyst of any one of claims 4 to 7, wherein the antimony-containing compound is selected from the group consisting of antimony trioxide ($Sb_2O_3$) and antimony pentoxide ($Sb_2O_5$).

9. The method for forming an oxidative dehydrogenation catalyst of any one of claims 4 to 8, wherein the alkylene glycol is ethylene glycol ($C_2H_6O_2$).

10. The method for forming an oxidative dehydrogenation catalyst of any one of claims 5 to 9, wherein the hydrothermal synthesis temperature is from 150 °C to 250 °C, such as from 180 °C to 220 °C, and more preferably 180 °C to 210 °C.

11. The method for forming an oxidative dehydrogenation catalyst of any one of claims 5 to 10, wherein the starting mixture comprises MoOs and $V_2O_5$.

12. A method for converting paraffins to olefins, the method comprising:

a) contacting a feed stream comprising paraffins with a material comprising the oxidative dehydrogenation catalyst of any one of claims 1 to 4;
b) converting at least a portion of the paraffins to olefins, thereby yielding a product stream comprising paraffins and olefins; and
c) separating the olefins from the paraffins in the product stream.

13. The method of claim 12, wherein the method further comprises contacting a second stream comprising oxygen with the feed stream comprising paraffins and the oxidative dehydrogenation catalyst.

14. The method of claim 13, wherein the conversion occurs at a temperature that is from 300 °C to 700 °C, and a pressure that is from 0 barg to 20 barg.

15. The method of any of claim 13 and claim 14, wherein the conversion occurs at an alkane weight hourly space velocity (WHSV) that is from 0.1/hr to 10/hr.

**Amended claims in accordance with Rule 137(2) EPC.**

1. An oxidative dehydrogenation catalyst comprising:

(i) a structure comprising a mixed oxide of molybdenum (Mo), vanadium (V), niobium (Nb), antimony (Sb) and bismuth (Bi) having a formula $MoV_{0.2-0.3}Nb_{0.005-0.02}Sb_{0.001-0.1}Bi_{0.05-0.15}O_x$, wherein x is an oxygen content required to charge-balance the structure; and

(ii) a crystallographic structure with the Pba2-32 space group, **characterized by** reflections determined with Cu-$K_\alpha$ X-ray diffraction (XRD) as follows:

| 2θ (± 0.3°) | Rel. Intensity (%) |
|---|---|
| 5.3 | 0.2 - 10 |
| 6.6 | 1.5 - 15 |
| 7.84 | 2.5 - 45 |
| 8.95 | 4 - 21 |
| 22.17 | 100 |
| 27.2 | 20 - 70 |

**2.** The oxidative dehydrogenation catalyst of claim 1, wherein the oxidative dehydrogenation catalyst is essentially free of tellurium (Te).

**3.** A method for forming an oxidative dehydrogenation catalyst, the method comprising:

a) adding a molybdenum-containing compound, a vanadium-containing compound, a bismuth-containing compound, a niobium-containing compound, an antimony-containing compound, and one or more organic acids to a mixture of alkylene glycol or alcohol amine and water to form a starting mixture;

b) treating the starting mixture by hydrothermal synthesis at a hydrothermal synthesis temperature that is from 150 °C to 250 °C; and

c) separating $Mo_uV_vNb_wSb_yBi_zO_x$ from retained liquids,

wherein the starting mixture comprises at least one of molybdenum trioxide (MoOs) and vanadium pentoxide ($V_2O_5$).

**4.** The method for forming an oxidative dehydrogenation catalyst of claim 5, wherein the one or more organic acids comprises at least one of citric acid ($C_6H_8O_7$), oxalic acid ($C_2H_2O_4$), and mixtures thereof.

**5.** The method for forming an oxidative dehydrogenation catalyst of any one of claim 3 or claim 4, wherein the niobium-containing compound is selected from the group consisting of niobium oxide, niobic acid ($Nb_2O_5\cdot xH_2O$), ammonium niobium oxalate hydrate($(NH_4)Nb(C_2O_4)_2.nH_2O$), niobium ethoxide, and mixtures thereof.

**6.** The method for forming an oxidative dehydrogenation catalyst of any one of claim 3 to 5, wherein the bismuth-containing compound is selected from the group consisting of bismuth oxide ($Bi_2O_3$), bismuth sulfate ($Bi_2(SO_4)_3$), bismuth citrate ($BiC_6H_5O_7$), and bismuth nitrate ($Bi(NO_3)_3$).

**8.** The method for forming an oxidative dehydrogenation catalyst of any one of claims 4 to 7, wherein the antimony-containing compound is selected from the group consisting of antimony trioxide ($Sb_2O_3$) and antimony pentoxide ($Sb_2O_5$).

**8.** The method for forming an oxidative dehydrogenation catalyst of any one of claims 3 to 7, wherein the alkylene glycol is ethylene glycol ($C_2H_6O_2$).

**9.** The method for forming an oxidative dehydrogenation catalyst of any one of claims 4 to 8, wherein the hydrothermal synthesis temperature is from 150 °C to 250 °C, such as from 180 °C to 220 °C, and more preferably 180 °C to 210 °C.

**10.** The method for forming an oxidative dehydrogenation catalyst of any one of claims 4 to 9, wherein the starting mixture comprises MoOs and $V_2O_5$.

**11.** A method for converting paraffins to olefins, the method comprising:

a) contacting a feed stream comprising paraffins with a material comprising the oxidative dehydrogenation catalyst of any one of claims 1 to 2;
b) converting at least a portion of the paraffins to olefins, thereby yielding a product stream comprising paraffins and olefins; and
c) separating the olefins from the paraffins in the product stream.

**12.** The method of claim 11, wherein the method further comprises contacting a second stream comprising oxygen with the feed stream comprising paraffins and the oxidative dehydrogenation catalyst.

**13.** The method of claim 12, wherein the conversion occurs at a temperature that is from 300 °C to 700 °C, and a pressure that is from 0 barg to 20 barg.

**14.** The method of any of claim 12 and claim 13, wherein the conversion occurs at an alkane weight hourly space velocity (WHSV) that is from 0.1/hr to 10/hr.

FIG. 1

Europäisches
Patentamt

European
Patent Office

Office européen
des brevets

# EUROPEAN SEARCH REPORT

**Application Number**

EP 23 38 2181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 524 236 A (MCCAIN JAMES H [US]) 18 June 1985 (1985-06-18) * column 3, line 47 – line 60; claims; examples; tables * * column 5, line 40 – line 50 * ----- | 1-15 | INV. B01J23/00 B01J23/28 B01J35/00 |
| X | US 6 486 091 B1 (ABDULWAHED MAZHAR [SY] ET AL) 26 November 2002 (2002-11-26) * example 1 * ----- | 1-3 | |
| E | WO 2023/028433 A1 (DOW GLOBAL TECHNOLOGIES LLC [US]) 2 March 2023 (2023-03-02) * claims; figures; examples; tables * ----- | 1-15 | |

**TECHNICAL FIELDS SEARCHED (IPC)**

B01J

The present search report has been drawn up for all claims

| Place of search | Date of completion of the search | Examiner |
|---|---|---|
| Munich | 9 August 2023 | de Cauwer, Robby |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 38 2181

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2023

| Patent document cited in search report | | Publication date | Patent family member(s) | | Publication date |
|---|---|---|---|---|---|
| US 4524236 | A | 18-06-1985 | AU | 587981 B2 | 07-09-1989 |
| | | | BR | 8503080 A | 11-03-1986 |
| | | | CA | 1229351 A | 17-11-1987 |
| | | | EP | 0167109 A2 | 08-01-1986 |
| | | | JP | H0354927 B2 | 21-08-1991 |
| | | | JP | S6115849 A | 23-01-1986 |
| | | | KR | 860000233 A | 27-01-1986 |
| | | | MX | 167427 B | 22-03-1993 |
| | | | NO | 160435 B | 09-01-1989 |
| | | | US | 4524236 A | 18-06-1985 |
| | | | YU | 108185 A | 31-10-1987 |
| | | | ZA | 854882 B | 26-02-1986 |
| US 6486091 | B1 | 26-11-2002 | DE | 60100265 T2 | 09-10-2003 |
| | | | EP | 1155741 A1 | 21-11-2001 |
| | | | JP | 4410954 B2 | 10-02-2010 |
| | | | JP | 2001259420 A | 25-09-2001 |
| | | | SA | 05260374 B1 | 25-09-2006 |
| | | | US | 6486091 B1 | 26-11-2002 |
| WO 2023028433 | A1 | 02-03-2023 | NONE | | |

EPO FORM P0459

For more details about this annex : see Official Journal of the European Patent Office, No. 12/82